# EUROPÄISCHE PATENTSCHRIFT

(11) **EP 0 749 435 B1**
(45) Veröffentlichungstag und Bekanntmachung des Hinweises auf die Patenterteilung: **11.10.2000**
(21) Anmeldenummer: 95912194.8
(22) Anmeldetag: 03.03.1995
(51) Int. Cl.: C07D 495/04, C07K 14/435, C07K 14/36, G01N 33/53

(54) **ENTSTÖRMITTEL ZUM EINSATZ BEI IMMUNOASSAYS**
INTERFERENCE SUPPRESSION AGENT FOR USE IN IMMUNO ASSAYING
PRODUIT DE SUPPRESSION DES INTERACTIONS DANS LES DOSAGES IMMUNOLOGIQUES

(30) Priorität: 05.03.1994 DE 4407423
(43) Veröffentlichungstag der Anmeldung: 27.12.1996
(73) Patentinhaber: Roche Diagnostics GmbH, 68298 Mannheim (DE)
(72) Erfinder: KIENTSCH-ENGEL, Rosemarie, D-82340 Feldafing (DE); DONIE, Frederic, D-82377 Penzberg (DE); WIEDMANN, Michael, D-82377 Penzberg (DE)
(86) Internationale Anmeldenummer: EP9500776
(87) Internationale Veröffentlichungsnummer: WO9523801

(56) Entgegenhaltungen:
- EP-A- 0 155 854
- EP-A- 0 331 068

## Beschreibung

Die Erfindung betrifft Avidin- oder Streptavidin oder ein Derivat hiervon als Entstörmittel in Immunoassays sowie Verfahren zum Nachweis eines Analyten unter Verwendung dieser Entstörmittel.

Immunologische Nachweismethoden haben in den letzten Jahren eine große Bedeutung erlangt. Mit ihnen kann die Gegenwart von Arzneimitteln, Hormonen, Proteinen, infektiösen Organismen und insbesondere spezifischer Antikörper in biologischen Proben schnell und genau nachgewiesen werden. Bei allen immunologischen Nachweismethoden kommt es zu einer spezifischen Bindungsreaktion zwischen einem ersten spezifischen Bindungspartner, der Substanz, die nachgewiesen werden soll ("Analyt") und einem zweiten spezifischen Bindungspartner, der spezifisch mit dem Analyten reagiert oder ihn bindet. Analyt und spezifischer Analytbindungspartner, die sogenannten Partner eines spezifischen Bindungspaares, bilden dabei ein spezifisches Bindungspaar, im allgemeinen ein Komplex zwischen einem Antigen und einem Antikörper oder Antikörperfragment. Dabei können mehr als ein Analyt oder ein Bindungspartner in jeder Reaktion miteinander reagieren. Diese spezifischen Bindereaktionen werden auf verschiedene Weise detektiert. Im allgemeinen ist ein Teilnehmer der spezifischen Bindereaktion markiert. Übliche Markierungsmethoden sind Radioisotope, Chromogene, Fluorogene, Enzymmarkierung oder Substanzen, die wiederum ein spezifisches Bindpaar bilden können (z.B. Biotin/Streptavidin). Bei heterogenen Immunoassays ist einer der Bindungspartner an eine Festphase immobilisiert.

Ein schwerwiegendes Problem bei Immunoassays ist, daß zwischen spezifischen Bindungspartnern des Immunoassays und der Probe, der in der Probe enthaltenen zusätzlichen Bestandteilen und gegebenenfalls der Festphase unerwünschte Wechselwirkungen und unspezifische Bindereaktionen erfolgen können. Derartige Wechselwirkungen bewirken im allgemeinen eine Erhöhung des Hintergrundsignals und auch eine stärkere Streuung der Signale und damit eine verringerte Sensitivität und Spezifität des betreffenden Testes. Sowohl durch unspezifische Wechselwirkung mit dem markierten Bindungspartner als auch durch spezifische Bindung von Testkomponenten durch Probenbestandteile können auch falsch-positive Messungen resultieren, das heißt, es wird auf Grund des falsch erhöhten Meßsignals auf die Anwesenheit eines Analyten auch bei dessen Abwesenheit geschlossen.

Es wurden verschiedene Versuche unternommen, diese unspezifischen Wechselwirkungen in Immunoassays zu reduzieren. Seit langem ist bekannt, daß unterschiedliche Kohlenhydratkomponenten und unterschiedliche Proteine, Proteingemische oder Proteinfraktionen sowie deren Hydrolysate unspezifische Wechselwirkungen zwischen den Testkomponenten und dem Analyten in Immunoassays reduzieren können (beispielsweise Robertson et al., Journal of Immun.Meth. 26, 1985, 195; EP-A-260903; US-A-4,931,385). Der Einsatz von Protein rohfraktionen und Rohhydrolysaten hat den Nachteil, daß durch die darin enthaltenen Bestandteile wiederum andere Störungen des Tests ausgelöst werden können. Enzymatisch produzierte Hydrolysate können zudem mit den bei der Herstellung verwendeten Proteasen kontaminiert sein und weisen in der Regel keine einheitliche Qualität auf, da sich die Spaltung nur schwer steuern läßt. Proteasekontaminationen können Testkomponenten angreifen und schon in geringen Mengen zur Beeinträchtigung der Testfunktionen und Lagerstabilität führen.

Zur Verringerung unspezifischer Wechselwirkungen in Immunoassays wurde auch der Einsatz von chemisch modifizierten Proteinen, insbesondere von succinylierten oder acetylierten Proteinen, beschrieben (US-A-5,051,356; EP-A-0 525 916). Mit diesen Substanzen können jedoch viele der falsch positiven Ergebnisse bei Tests auf Antikörper aus Serum nicht vermieden werden.

EP-A-0 331 068 und WO 91/06559 beschreiben den Einsatz von polymerisiertem Immunoglobulinen, insbesondere IgG, zur Reduktion spezifischer Störfaktoren wie z.B. Rheumafaktoren. Es lassen sich damit aber nicht alle störenden Wechselwirkungen zufriedenstellend ausschalten. Außerdem kann der Zusatz von unspezifischen humanem Immunglobulin (monomere oder polymere Antikörper oder deren Fragmente) in Tests auf humane Antikörper zu einer Erhöhung des Leerwertes führen. Ferner ist die Gewinnung von humanem oder tierischem IgG aufwendig und teuer.

Aufgabe der Erfindung war es daher, neue Entstörsubstanzen bzw. neue Entstörmittel zur Verfügung zu stellen, die eine bessere Entstörung von unspezifischen Wechselwirkungen bei Immunoassays bewirken, als aus dem Stand der Technik bekannt. Unter unspezifischen Wechselwirkungen sind alle Wechselwirkungen zwischen Komponenten des Verfahrens zu verstehen, die zu Verfälschungen des Meßergebnisses führen können. Die Entstörsubstanzen sollten falsch-positive Analysenergebnissen vermeiden, besonders bei der Analyse von Antikörpern. Insbesondere soll eine Störung bei der Verwendung von Avidin- oder Streptavidin als ein Bindepartner in einem Immunoassay vermieden werden.

Gelöst wurde diese Aufgabe durch Avidin- oder Streptavidin oder deren Derivate als Entstörmittel. Durch die Verwendung dieser Substanzen konnte überraschenderweise eine Entstörung bei Assays insbesondere bei Assays, bei denen Avidin- oder Streptavidin als ein Bindepartner eingesetzt wird, erzielt werden.

Gegenstand der Erfindung sind daher Entstörmittel zur Vermeidung von unspezifischen Wechselwirkungen in Assays, die Avidin- oder Streptavidin oder deren Derivate enthalten und die Verwendung dieser Entstörmittel in Assays. Avidin- oder Streptavidin oder deren Derivate werden erfindungsgemäß in löslicher Form eingesetzt. Sie sind nicht an eine feste Phase oder eine Markierungsgruppe beispielsweise ein Enzym gebunden bzw. gekoppelt. Das erfindungsgemäße Entstörmittel wird immer zusätzlich zu den ansonsten für den Test notwendige Reagenzien eingesetzt und wird kein Bestandteil des sich im Laufe der Nachweisreaktion bildenden Komplexes aus nachzuweisendem Analyt und spezifischen Bindepartnern. Bevorzugt wird das erfindungsgemäße Entstörmittel bei immunologischen Tests (Immunoassays) eingesetzt. Es kann aber auch bei Tests verwendet werden, die andere Wechselwirkungen zur Grundlage haben, beispielsweise Nukleinsäuretests. Bevorzugt wird das Entstörmittel bei Tests verwendet, bei denen eine Testkomponente an Avidin oder Streptavidin gebunden ist, beispielsweise eine Avidin- oder Streptavidinbeschichtete Festphase bei Immuno- oder Nukleinsäuretests. Unter Avidin- oder Streptavidin sind die natürlich vorkommenden, gereinigten Proteine oder rekombinantes Avidin oder Streptavidin zu verstehen. Unter Avidin- oder Streptavidinderivaten sind modifizierte Avidin- oder Streptavidinmoleküle zu verstehen. Die Modifikation kann erstens durch Vernetzung einzelner Avidin- oder Streptavidinmoleküle erfolgen, so daß sogenanntes poly SA oder homogen vernetztes SA entsteht. Unter SA ist im folgenden immer Avidin oder Streptavidin zu verstehen. Methoden zur Vernetzung oder Polymerisierung von SA sind dem Fachmann an sich bekannt. Insbesondere ist eine Vernetzung durch Hitzebehandlung oder durch bi- oder polyfunktionelle Verbindungen geeignet. Unter bi- oder polyfunktionellen Verbindungen werden Moleküle verstanden, die mindestens zwei funktionelle Gruppen tragen, die gleich oder verschieden sein können und über diese funktionellen Gruppen mit funktionellen Gruppen von SA beispielsweise bestimmte Aminosäurereste oder Kohlehydratreste reagieren können. Typische Beispiele für im Rahmen der Erfindung geeignete Linker sind in der folgenden Tabelle 1 aufgeführt.

**Tabelle 1**

| **Kurzbezeichnung** | **Chemische Zusammensetzung** |
|---|---|
| SPDP | N-succinimidyl 3-(2-pyridyldithio)-propionat |
| EADP | Ethyl 4-azidohenyl-1,4-dithiobutyrimidat · HCl |
| FNPA | 4-Fluoro-3-nitrophenylazid |
| HSAB | N-Hydroxysuccinimidyl-4-azidobenzoat |
| MABI | Methyl-4-azidobenzoimidat · HCl |
| MBS | m-Maleimidobenzoyl-N-Hydroxysuccinimidester |
| NHS-ASA | N-Hydroxysuccinimidyl-4-azidosalicylsäure |
| MHS | Maleimidohexanoyl-N-Hydroxysuccinimidester |
| PNP-DTP | p-Nitrophenyl-2-diazo-3,3,3-trifluoropropionat |
| SADP | N-Succinimidyl(4-azidophenyl)1,3'-dithiopropionat |
| SAND | Sulfosuccinimidyl-2-(m-azido-o-nitobenzamido)ethyl-1,3'-dithiopropionat |
| SANPAH | N-succinimidyl-6(4'-azido-2'-nitrophenyl-amino)hexanoat |
| SASD | Sulfosuccinimidyl 2-(p-azidosalicylamido)ethyl-1,3'-dithiopropionat |
| SIAB | N-Succinimidyl(4-iodoacetyl)aminobenzoat |
| SMCC | Succinimidyl-4-(N-maleinimidoethyl)cyclohexan-1-carbxylat |
| SMPB | Succinimidyl-4-(p-maleinimidophenyl)butyrat |
| DSS | Disuccinimidylsuberat |
| DMS | Dimethylsuberimidat |
| Traut's Reagenz | 2-Iminothiolan 2,4,6 Trichlor-s-thiazin |
| SAMBA | S'-Acetyl-mercaptobernsteinsäureanhydrid |
| SATP | N-Succinimidyl-S-Acetylthiopropionat |
| SATA | N-Succinimidyl-S-Acetylthioacetat |

Zweitens kann die Modifikation durch eine Kopplung an andere hochmolekulare Moleküle insbesondere Proteine wie Rinderserumalbumin (RSA) oder Immunglobulin, die oft selbst zur Entstörung in Immunoassays eingesetzt werden, bestehen. Durch diese Vernetzung entsteht sogenanntes heterogen vernetztes SA. Dabei können in diesem Komplex SA und/oder das Protein wiederum polymerisiert sein. Methoden zur Kopplung von SA an diese Moleküle sind dem Fachmann bekannt. Insbesonders geeignet erwiesen hat sich eine Kopplung mit DSS, SAMBA, SATP, MHS, SATA. Ein Komplex aus RSA und SA oder poly SA ist als Entstörmittel besonders geeinet. Noch bessere Effekte werden erzielt, wenn polymerisiertes RSA an SA oder poly SA gekoppelt wird. Die Polymerisierung oder Vernetzung von RSA kann mit den ober genannten Methoden zu Vernetzung von SA erfolgen. Am geeignetsten ist eine Polymerisierung durch Hitzebehandlung wie in der EP-A 0331 127 beschrieben.

Drittens kann die Modifikation von SA durch eine Inaktivierung der aktiven Zentren, d. h. der Bindestellen für Biotin, bestehen. Hierdurch entsteht sogenanntes inaktiviertes SA. Die Inaktivierung kann durch einfache Absättigung der Bindestellen mit Biotin oder einem Biotinderivat, das von Avidin- oder Streptavidin gebunden wird, erfolgen. Durch die sehr hohe Affinität von SA zu Biotin wird beim Einsatz im Immunoasaay kaum Biotin freigesetzt, was eventuell zu Störungen führen könnte. Bevorzugt wird das aktive Zentrum kovalent modifiziert. Dabei können eine oder mehrere Aminosäurereste im aktiven Zentrum von SA derivatisiert werden, sodaß die Bindung an Biotin stark vermindert oder ganz verhindert wird. Methoden zur Inaktivierung der Biotinbindestelle in SA sind aus Gitlin et al., Biochem. J. 269 (1990) 527-530 bekannt. Bevorzugt werden Tyrosin-Reste im aktiven Zentrum von SA mit p-Nitrobenzolsulfonylfluorid modifiziert. Eine weitere Möglichkeit zur Inaktivierung der Biotinbindestelle in SA besteht darin, Biotin an der Bindestelle kovalent zu koppeln. Methoden zur Kopplung von Biotin an das aktive Zentrum von SA sind dem Fachmann bekannt. Als besonders bevorzugt hat sich eine neue Methode zur Kopplung von Biotin an SA mittels eines neuen photoaktivierbaren Biotinderivats herausgestellt. Photoaktivierbare Biotinderivate sind bekannt. In EP-A-0 155 854 und EP-A-0 187 323 werden azidsubstituierte Phenyle/Nitrophenyle beschrieben, die mit einem aminhaltigen Linker an Biotin gekoppelt sind. Als Biotinderivat wird bevorzugt Biotin-DADOO-AB (Biotin-[8-(4-azidobenzoyl)amino-3,6-dioxaoctyl]amid) verwendet. Weitere Biotinderivate werden im Boehringer Mannheim Biochemica Katalog, Ident Nr. 1292633 bzw. 1292641 beschrieben. Nach der Absättigung der Biotinbindestellen von SA mit den Biotinderivaten wird die Photoreaktion initiiert und so Biotin kovalent am aktiven Zentrum fixiert. Bei diesem inaktiviertem SA ist Biotin an das aktive Zentrum und zusätzlich über eine kovalente Bindung außerhalb des aktiven Zentrums gebunden. Das hierdurch erhältliche SA-Biotin-Produkt ist ebenfalls Gegenstand der vorliegenden Erfindung.

Eine weitere Möglichkeit zur Inaktivierung des aktiven Zentrums von SA besteht darin, die Bindesteilen gentechnologisch zu verändern. Das aktive Zentrum von SA kann durch Substitution, Deletion oder Insertion einzelner Aminosäurereste oder kurzer Abschnitte von Aminosäureresten modifiziert und inaktiviert werden. Bevorzugt können einzelne Aminosäuren, beispielsweise die Tyrosinreste, im aktiven Zentrum gegen andere Aminoäuren ausgetauscht werden. Es kann aber auch SA hergestellt werden, bei dem die Bindestelle teilweise oder vollständig fehlt. Methoden zur gentechnischen Modifikation und Herstellung von SA sind dem Fachmann bekannt. Beispielsweise ist die Herstellung von rekombinantem Streptavidin in der EP-A-0 198 015 beschrieben.

Viertens kann die Modifikation durch eine Fragmentierung von SA erreicht werden. Die SA-Fragmente können duch chemische oder enzymatische Spaltung oder durch rekombinante Herstellung erzeugt werden. Hierbei kann insbesondere die Biotin-Bindungsstelle, wie oben bereits beschrieben, fehlen. Vorteil der Fragmentierung ist eine bessere Löslichkeit des Produktes. Bevorzugt werden alle durch chemische oder enzymatische Spaltung aus Avidin oder Streptavidin erhältlichen Fragmente im Gemisch eingesetzt, sodaß alle oder alle Teile aus SA enthalten sind.

Das auf diese Weise inaktivierte oder fragmentierte SA kann in der oben genannten ersten oder zweiten Methode zu Modifizierung von SA eingesetzt werden, d. h. das inaktivierte SA kann wiederum polymerisiert oder mit anderen Molekülen wie RSA oder poly RSA vernetzt werden. Diese Entstörmittel, bei denen eine Kombination von mindestens zwei der genannten Methoden zur Modifikation von SA durchgeführt wurde, haben sich als besonders vorteilhaft bewiesen.

Nach der erfolgten Modifikation von SA durch eine der genannten Methoden kann eventuell verbliebene Biotin-Bindeaktivität kovalent an der Oberfläche gebundenes Biotin, das nicht im aktiven Zentrum von Avidin oder Streptavidin gebunden ist, oder freies Biotin durch eine geeignete Anfreinigung der Avidin- oder Streptavidinderivate entfernt werden. Dies kann beispielsweise über eine Adsorption an festphasengebundenes Biotin und/oder SA geschehen.

Gegenstand der Erfindung sind daher weiterhin Avidin- oder Streptavidinderivate, erhältlich durch eine der oben beschriebenen Verfahren zur Modifikation und anschließender Aufreinigung zur Entfernung verbliebener Biotin-Bindeaktivität, kovalent an der Oberfläche gebundenem Biotin, das nicht im aktiven Zentrum von Avidin oder Streptavidin gebunden ist, oder freiem Biotin bevorzugt durch eine Adsorption an festphasengebundenem Biotin und/oder SA.

Gegenstand der Erfindung ist ferner ein Verfahren zur Herstellung der erfindungsgemäßen Entstörmittel, in dem SA nach mindestens einer der drei oben genannten Methoden modifiziert und gegebenenfalls anschließend zur Entfernung verbliebener Biotin-Bindekapazität, kovalent an der Oberfläche gebundenem Biotin oder freiem Biotin, wie zuvor beschrieben, aufreinigt.

Avidin- oder Streptavidin oder deren Derivate können zur Entstörung in allen gängigen Immunoassays oder Nukleinsäureassays eingesetzt werden. Besonders geeignet sind sie zur Entstörung von Immunoassays oder Nukleinsäureassays, bei denen als eine Bindekomponente Avidin- oder Streptavidin eingesetzt wird. Solche Immunoassays sind beispielsweise aus Guesdon et al., J. Histochem. Cytochem. 27 (1979) 1131-1139 und Bayer und Wilchek, Analytical Biochemistry 171 (1988) 1-32 bekannt. Die Störungen können beispielsweise durch Antikörper gegen Avidin oder Streptavidin, die teilweise im humanem Serum vorkommen, verursacht werden. Aber auch in Immunoassays, bei denen kein Avidin oder Streptavidin eingesetzt wird, zeigt das erfindungsgemäße Entstörmittel eine vorteilhafte Wirkung. In diesen Immunoassays hat sich die Verwendung von SA, das an ein weiteres Molekül gebunden ist wie beispielsweise RSA oder polyRSA, entsprechend der ober beschriebenen zweiten Methode zur Modifikation, als besonders vorteilhaft erwiesen. Das erfindungsgemäße Entstörmittel wird immer zusätzlich zu den ansonsten im Test notwendigen Reagenzien eingesetzt. Es ist nicht identisch mit den möglicherweise im Test vorkommenden Avidin- oder Streptavidin-Komponenten, beispielsweise SA-beschichtete Festphase oder Enzym-SA-Konjugate. Das erfindungsgemäße Entstörreagenz wird im Gegensatz zu diesen SA-Reagenzien nicht in den nachzuweisenden Komplex aus Analyt und spezifischen Bindepartnern eingebaut.

Ein weiterer Gegenstand der Erfindung ist ein Verfahren zur Bestimmung eines Analyten in einer Probe durch
(1) Kontaktieren der Probe mit
   (a) Avidin- oder Streptavidin oder einem Derivat hiervon,
   (b) einem oder mehreren spezifischen Bindepartnern des Analyten und
(2) Messung des gebildeten Komplexes aus Analyt und spezifischem Bindepartner als ein Maß für die Anwesenheit des Analyten.

Als Analyt können alle Substanzen dienen, die mit mindestens einem spezifischen Bindepartner spezifisch zu einem Komplex reagieren wie zum Beispiel Haptene, Antigene, Antikörper oder Nukleinsäuren. Bei dem Nachweis von Antikörpern insbesondere Autoantikörper ist das erfindungsgemäße Verfahren besonders geeignet.

Als Probe dienen im allgemeinen Körperflüssigkeiten wie Blut, Plasma, Serum, Speichel oder Urin.

Als spezifischer Bindepartner kann jeder biologische oder chemische Bindungspartner dienen, der spezifisch den Analyten zu binden vermag und mit diesem einen Komplex ausbilden kann. Hierzu zählen Antikörper, Antikörperfragmente, Antigen, Hapten, Hormone, Avidin, Biotin, Nukleinsäuren, Oligonukleotide oder Derivate davon. Bevorzugt werden in der vorliegenden Erfindung als Bindepartner des Analyten Antikörper oder Antigene oder deren Fragmente eingesetzt.

Zum Nachweis des Komplexes aus Analyt und spezifischem Bindepartner können alle dem Fachmann gängigen Methoden eingesetzt werden. Es können homogene Verfahren, bei denen alle Bindepartner im Verfahren in löslicher Form vorkommen beispielsweise Präzipitationsverfahren mit turbidimetrischer oder nephelometrischer Bestimmung des gebildeten Komplexes oder Immunoassays nach dem CEDIA-, EMIT- oder FPIA-Prinzip verwendet werden. Geeignet sind ebenfalls heterogene Verfahren bei denen mindestens ein Reagenz an eine feste Phase gebunden ist. Beispiele hierfür sind Agglutinationstests, bei denen ein Partner eines Bindepaares beispielsweise an Latex gebunden ist, Sandwichassays, ELISA min Nachweis von spezifischen Antikörpern wie z.B. gegen HIV, HCV, Rubella, Toxoplasma gondii, Glutamatdecarboxylase oder Thyreoglobulin, RIA oder immunometrische Assays. Außer bei dem Präzipitationsverfahren ist in allen diesen Verfahren mindestens einer der spezifischen Bindepartner markiert. Die Markierung kann direkt ein meßbares Signal liefern beispielsweise ein Radioisotop, ein Chemilumineszenz-, Fluoreszenz- oder Elektrochemilumineszenz-Marker oder ein gefärbter Partikel beispielsweise ein Metallsolpartikel oder gefärbter oder ungefärbter Latex. Die Markierung kann auch ein indirektes Signal liefern, beispielsweise eine Enzymmarkierung wie Peroxidase, Glucoseoxidase, ß-Galaktosidase oder alkalische Phosphatase.

Die Immunoassays können ebenfalls mittels Teststreifen oder Biosensoren durchgeführt werden, insbesondere wenn einer der Bindepartner über SA an die Festphase gekoppelt ist. Auch Immunoassays nach dem Prinzip der Plasmonresonanz können erfindungsgemäß entstört werden.

Oft wird ein Reagenz des Verfahrens zum Nachweis des Analyten über ein spezifisch bindendes Bindepaar beispielsweise Avidin bzw. Streptavidin/Biotin an die Festphase gekoppelt. Der Vorteil hierbei ist, daß die Festphase universell bei mehreren Nachweisverfahren eingesetzt werden kann. Auch ist es möglich die Markierung über ein spezifisches Bindepaar an eine Komponente des Assays zu binden. Beispielsweise kann ein Enzym an Avidin oder Streptavidin gekoppelt sein und der Bindepartner beispielweise ein Antikörper kann biotinyliert sein. Beispiele für solche Nachweisverfahren sind dem Fachmann bekannt. Das erfindungsgemäße Avidin- oder Streptavidinderivat ist zur Entstörung dieser Nachweisverfahren, die eine indirekte Bindung einer Reaktionskomponente über Avidin/Biotin bzw. Streptavidin/Biotin nutzen besonders geeignet.

Die Durchführung des Verfahrens zum Nachweis des Analyten kann in einem oder mehreren Schriften erfolgen, d. h. die Inkubation des Analyten mit den einelnen Testkomponenten kann gleichzeitig oder nacheinander erfolgen. Wird Avidin- oder Streptavidin oder ein Derivat hiervon benutzt, bei dem die Biotin-Bindungsstellen nicht inaktiviert wurden, so muß bei Verfahren, bei denen eine Bindekomponente über Avidin/Biotin bzw. Streptavidin/Biotin gekoppelt wird, darauf geachtet werden, daß die Bindung der Bindekomponente über Avidin/Biotin bzw. Streptavidin/Biotin bereits abgeschlossen ist, bevor Avidin- oder Streptavidin oder ein Derivat hiervon zugesetzt wird, da anderenfalls die nicht inaktivierten Biotin-Bindestellen die biotinylierte Bindekomponente binden und Störungen hervorrufen. Beispielsweise muß bei der Benutzung einer Avidin- oder Streptavidin-Festphase zunächst der biotinylierte spezifische Bindepartner des Analyten zugegeben werden, bevor die Probe zusammen mit Avidin- oder Streptavidin oder deren Derivat zugegeben wird. Falls ein erfindungsgemäßes Avidin- oder Streptavidinderivat benutzt wird, bei dem die Biotin-Bindestellen inaktiviert wurden, können alle Testreagenzien simultan inkubiert werden, da die biotinylierten Reagenzien nicht mit dem Avidin- oder Streptavidinderivat binden. Dieses inaktivierte Avidin- oder Streptavidinderivat ist somit in allen denkbaren Testvarianten universell einsetzbar und damit bevorzugt.

Die Konzentration des erfindungsgemäßen Entstörmittels im Testgemisch, liegt zwischen 0,0001 und 1 % (m/v), bevorzugt zwischen 0,01 und 1 % (m/v).

Die einzelnen Reaktionskomponenten des Verfahrens zum Nachweis des Analyten werden zweckmäßigerweise in Form einer Testkombination oder eines Testkits angeboten. Ein weiterer Gegenstand der Erfindung ist daher eine Testkombination für ein Verfahren zum Nachweis eines Analyten in einer Probe enthaltend Avidin- oder Streptavidin oder ein Derivat hiervon und mindestens einen spezifischen Bindepartner des Analyten. Weiterhin können alle weiteren zur Durchführung des Nachweisverfahrens notwendigen Reagenzien wie Puffer, Detergenzien, Markierung, Hilfsstoffe zum Nachweis der Markierung wie Enzymsubstrate, Festphasen usw. enthalten sein. Bevorzugt wird das erfindungsgemäße Entstörmittel und der oder die Bindepartner des Analyten in getrennten Behältnissen abgepackt. Falls ein erfindungsgemäßes Entstörmittel verwendet wird, bei die Biotin-Bindestelle inaktiviert wurde, kann das Entstörmittel auch zu den Bindepartnern des Analyten direkt zugesetzt werden.

Die Erfindung wird durch die folgenden Beispiele verdeutlicht:

### Beispiel 1

### Herstellung von polymerem Streptavidin

Die Herstellung von polymerisiertem Streptavidin erfolgte nach EP-A-0 331 127.

### Aktivierung von Streptavidin mit Maleimido-hexanoyl-N-hydroxysuccinimidester

30 mg Streptavidin werden in 3 ml 30 mM Kaliumphosphat/100 mM Natriumchlorid (pH 7,1) gelöst und auf 25°C temperiert. Unter Rühren werden 0,15 ml Maleinimido-hexanoyl-N-hydroxysuccinimidester (MHS)(Boehringer Mannheim GmbH) in DMSO (10 mg/ml) zugetropft. Nach einer Reaktionszeit von 1 Stunde bei 25°C wird die Lösung im Eisbad abgekühlt. Anschließend wird das entstandene MHS-Streptavidin bei 4°C zweimal gegen 1 Liter 50 mM Kaliumphosphat/100 mM Natriumchlorid (pH 5,0) dialysiert.

### Aktivierung von Streptavidin mit S-Acetylmercaptobernsteinsäureanhydrid

30 mg Streptavidin werden in 3 ml 100 mM Kaliumphosphat (pH 7,8) gelöst und auf 25°C temperiert. Unter Rühren werden 0.175 ml S-Acetylmercaptobernsteinsäureanhydrid (SAMBA) in DMSO (10 mg/ml) zugetropft. Nach einer Reaktionszeit von 3 Stunden bei 25°C wird das entstandene SAMBA-Streptavidin bei 4°C zweimal gegen 1 Liter 50 mM Kaliumphosphat/2 mM EDTA (pH 6,5) dialysiert.

### Homogene Vernetzung von Streptavidin

3 ml einer Lösung von aktiviertem SAMBA-Streptavidin (10 mg/ml) werden auf 25°C temperiert und mit 50 µl 1 M Hydroxylamin (pH 6,5) versetzt. Nach 30 Minuten bei 25°C wird durch Zugabe von 15 ml 50 mM Kaliumphosphat/100 mM Natriumchlorid/1 mM EDTA (pH 6,5) verdünnt. Die homogene Vernetzung des Streptavidins wird durch Zugabe von 3 ml aktiviertem MHS-Streptavidin (10 mg/ml) gestartet. Nach einer Reaktionszeit von 2 Stunden bei 25°C unter vorsichtigem Rühren wird die Reaktion durch Zugabe von 0,2 ml 100 mM Cystein/HCl beendet. Nach einer Inkubationszeit von 30 Minuten bei 25°C wird der pH-Wert der Lösung durch Zugabe von 1 M Dikaliumhydrogenphosphat auf 7,5 eingestellt. Nach Zugabe von 0,2 ml 500 mM Jodacetamid wird eine weitere Stunde bei 25°C inkubiert. Anschließend wird bei 4°C zweimal gegen 3 Liter 50 mM Kaliumphosphat/100 mM Natriumchlorid (pH 7,5) dialysiert. Das Konjugat wird nach der Dialyse in einer Ultrafiltrationszelle aufkonzentriert und nach Zusatz von Saccharose (8%) lyophilisiert.

### Beispiel 2

### Herstellung von Thermo-RSA-SA

Die Herstellung von Thermo-RSA-Streptavidin (Thermo-RSA-SA) erfolgte nach EP-A-0 331 127

### RSA-Vernetzung zu Thermo-RSA

1,0 g RSA werden in 100 ml 20 mM Kaliumphosphat-Puffer pH 7,0 gelöst und 5 Stunden bei einer Temperatur von 70°C gehalten. Anschließend wird auf 20°C abgekühlt und gegen 100 mM Kaliumphosphat-Puffer pH 7,8 dialysiert.

### Aktivierung von Thermo-RSA mit SAMBA

68 mg Thermo-RSA werden in 2 ml 0,1 M Kaliumphosphat-Puffer pH 7.8 gelöst und langsam mit 0,38 ml SAMBA (10 mg/ml in DMSO) versetzt. Nach einer Reaktionszeit von 3,5 Stunden bei 25°C wird bei 4°C gegen 1 Liter 50 mM Kaliumphosphat-Puffer pH 6,5 dialysiert.

### Herstellung des Thermo-RSA-Streptavidin-Konjugats

Die heterogene Vernetzung von Streptavidin mit Thermo-RSA erfolgt analog der unter dem Beispiel 1 beschriebenen homogenen Vernetzung. Dabei werden 60 mg aktiviertes MHS-Streptavidin (Herstellung nach Beispiel 1) mit 68 mg SAMBA-Thermo-RSA umgesetzt. Das Reaktionsprodukt wird durch Gelfiltration (Superose 6 prep. grade) aufgereinigt und in einer Ultrafiltrationszelle aufkonzentriert. Das erhaltene Produkt wird anschließend lyophilisiert.

### Beispiel 3

### Absättigung von Thermo-RSA-SA mit freiem Biotin

60 mg Thermo-RSA-SA gelöst in 6.0 ml Kaliumphosphat-Puffer, 100 mM, pH 7,0, werden mit 2 mg D-Biotin, gelöst in 0,5 ml Kaliumphosphat-Puffer, 10 mM, pH 7,8, versetzt und 1 Stunde gerührt.

Das freie Biotin wird über Gelfiltration (Superose 6®) abgetrennt. Die hochmolekulare Proteinfraktion wird gegen 10 mM Kaliumphosphat-Puffer, pH 7,0, dialysiert und nach Zugabe von 8% Saccharose lyophilisiert.

### Beispiel 4

### Herstellung von kovalent modifiziertem Streptavidin

Tyrosin-Reste im aktiven Zentrum von Streptavidin/Avidin werden mit p-Nitro-benzolsulfonylfluorid nach G. Gitlin, E. A. Bayer, M. Wilchek: Studies on the biotin-binding sites of Avidin and Streptavidin, Biochem. J. (1990), 269, 527-530 derivatisiert.

Zu 1 g Streptavidin wird bei einer Proteinkonzentration von 10 mg/ml in 0,1 M Tris-HCl-Puffer, pH 7,9 ein 200fach molarer Überschuß von p-Nitro-benzolsulfonylfluorid (Sigma N-2262) bezogen auf Streptavidin-Untereinheit zugesetzt. Nach Zugabe wird 18-20 Stunden bei 25° C weitergerührt. Das Produkt wird dann zur Abtrennung von nicht umgesetztem Derivatisierungsreagenz gegen >500faches Volumen PBS-Puffer pH 7,5 dialysiert (16-18 Stunden bei 4° C).

### Beispiel 5

### Herstellung von photoaktivierbarem Biotin Synthese von Biotin-[8-(4-azidobenzoyl)amino-3,6-dioxaoctyl]amid (Biotin-DADOO-AB)

1,50 g (4 mmol) Biotinoyl-1,8-diamino-3,6-dioxaoctan (Biotin-DADOO, Boehringer Mannheim GmbH ) werden unter Rühren in 50 ml frisch destilliertem DMF gelöst. Zu der Lösung gibt man nacheinander 1,04 g (4 mmol) N-Hydroxysuccinimidyl-(4-azidobenzoat) (HSAB, Boehringer Mannheim GmbH) und 0,55 ml (4 mmol) Triethylamin und läßt 2 Stunden bei 20°C rühren. Anschließend wird das Lösungsmittel am Rotationsverdampfer unter Ölpumpenvakuum entfernt und das verbleibende Rohprodukt durch Chromatographie an Kieselgel aufgereinigt. Hierzu wird in möglichst wenig Chloroform/Methanol 2/1 (v/v) unter leichtem Erwärmen auf ca. 40°C gelöst und auf eine Kieselgel 60 (Fa. Merck, BR Deutschland) Säule (4 x 60 cm) aufgetragen. Man eluiert mit Chloroform/Methanol 2/1 (v/v) und sammelt in 50 ml Fraktionen. Die das reine Produkt enthaltenden Fraktionen werden durch DC (System wie unten beschrieben) ermittelt und vereinigt. Das Lösungsmittel wird am Rotationsverdampfer entfernt und der halbfeste Rückstand mit ca. 50 ml Diisopropylether digeriert. Das feinkristalline, farblose Produkt wird abgesaugt und über Nacht im Vakuumtrockenschrank (0.1-0.15 bar/40°C) getrocknet.
- Ausbeute:: 1.24 g (60% d.Th.)
- DC:: Kieselgel 60(Merck) F₂₅₄, Chloroform/Methanol 2/1 (v/v); R_{f}= 0.71.
- ¹H-NMR(100MHz/d₆-DMSO):: δ(ppm) = 1.20-1.65(m, 6H); 2.07(tr, 2H); 2.60-3.65 (m, 15H); 4.05-4.20 (m; 2H); 6.38(d,br, 2H), 7.20 (d, 2H), 7.62 (tr,br, 1H); 7.91(d, 2H); 8.53 (tr,br, 1H).
- UV(CH₃OH):: λ(max) = 267nm
- IR(KBr):: ν = 2125 cm⁻¹

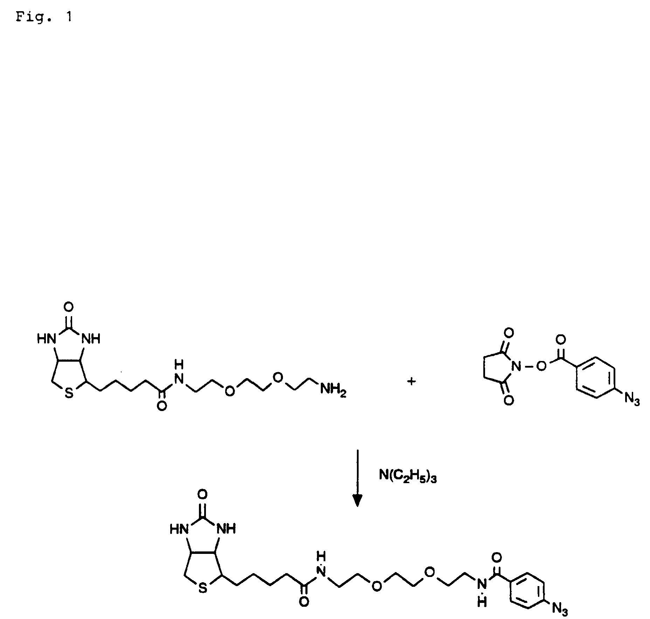

Der Syntheseweg von Biotin-DADOO-AB ist in der Figur 1 dargestellt.

### Beispiel 6

### Herstellung von Biotin(photoaktiviert)-Streptavidin

Streptavidin wird mit einem photoaktivierbarem Biotinderivat (z.B. Biotin-DADOO-AB) umgesetzt und zur Abtrennung des freien, nicht gebundenen Biotins dialysiert. Durch Bestrahlen mit Hg-Dampf-Lampe (350-700 nm) wird die Photoreaktion initiiert und das Biotin kovalent im Bindezentrum von Streptavidin fixiert.

Zu 1 g Streptavidin wird bei einer Proteinkonzentration von 20 mg/ml in PBS-Puffer pH 7,5 ein 10-facher molarer Überschuß von Biotin-DADOO-AB-Reagenz zugesetzt (3,5 ml einer 25 mg/ml Biotin-DADOO-AB-Stammlösung in DMSO). Nach Zugabe wird 2 Stunden bei 25°C unter Lichtausschluß gerührt.

Freies, nicht gebundenes Biotinderivat wird durch Dialyse (20 Std, 4°C) gegen >500faches Volumen PBS-Puffer, pH 7,5 unter Lichtausschluß vollständig (nicht mehr nachweisbar) abgetrennt. Der Ansatz wird dann bei einer Schichtdicke der Lösung von <5 cm mit einer Hg-Dampf-Lampe (350-700 nm) unter Rühren für 20 min bestrahlt und anschließend erneut gegen >500 faches Volumen PBS-Puffer, pH 7,5 dialysiert (16-18 Stunden, 4°C).

### Beispiel 7

### Reinigung von inaktiviertem Streptavidin bzw. Thermo-RSA-SA, bzw. deren Derivate und Fragmente

### Herstellung von RSA-Biotin-Adsorber

Zu 1 g RSA wird bei einer Proteinkonzentration von 10 mg/ml in PBS-Puffer pH 8,5 ein 10-facher molarer Überschuß von D-Biotinoyl-ε-aminocapronsäure-N-hydroxysuccinimidester (Boehringer Mannheim GmbH) zugesetzt.

Nach Zugabe wird 2 Stunden bei 25°C gerührt und die Reaktion durch Zugabe von Lysin auf eine Endkonzentration von 10 mM gestoppt. Freies, nicht gebundenes Biotinderivat wird durch Dialyse (16-18 Std, 4°C) gegen >500faches Volumen PBS-Puffer, pH 7,5 vollständig (nicht mehr nachweisbar) abgetrennt.

Zu 40 g Amino-Spherosil® (Boehringer Mannheim GmbH) werden 300 ml Gluthardialdehyd (10%ig) gegeben und der Ansatz 2 Stunden bei pH 3,7 und 55°C unter Rotieren gerührt. Die Suspension wird mit >7 Spherosil® -Volumina bidest. Wasser und 5 Spherosil®-Volumina PBS-Puffer, pH 8,0 gewaschen. Das aktivierte Spherosil® wird anschließend 20 Std. bei Raumtemperatur bei einem Proteinangebot von 5-10 mg/ml Spherosil mit RSA-Bi unter Schütteln umgesetzt.

Die nicht umgesetzte Proteinlösung wird über eine Glasfilternutsche abgetrennt und das Adsorbermaterial mit 10 Spherosil®-Volumina 0,9 %iger NaCl-Lösung gewaschen sowie mit 5 Spherosil® Volumina Äthanolamin-Lösung 1 Std. inkubiert.

Das Adsorbermaterial wird dann mit dem 5fachen Spherosil®-Volumen 0,9 %iger NaCl-Lösung, 3fachem Spherosil® Vol. 1M Propionsäure und mit genügend 30 mM NaCl-Lösung gewaschen, bis pH 6,5 erreicht ist. Der Adsorber wird in PBS-Puffer pH 7,5 ausreichend äquilibriert.

### Herstellung von Streptavidin-Spherosil-Adsorber

Zu 40 g Amino-Spherosil (Boehringer Mannheim GmbH) werden 300 ml Gluthardialdehyd (10%ig) gegeben und der Ansatz 2 Stunden bei pH 3,7 und 55°C unter Rotieren gerührt. Die Suspension wird mit >7 Spherosilvolumina bidest Wasser und 5 Spherosilvolumina PBS-Puffer, pH 8,0 gewaschen. Das aktivierte Spherosil wird anschließend 20 Std. bei RT bei einem Proteinangebot von 5-10 mg/ml Spherosil mit Streptavidin (Boehringer Mannheim GmbH) unter Schütteln umgesetzt.

Die nicht umgesetzte Proteinlösung wird über eine Glasfilternutsche abgetrennt und das Adsorbermaterial mit 10 Spherosilvolumina 0,9 %iger NaCl-Lsg gewaschen sowie mit 5 Vol. Äthanolamin-Lösung 1 Std. inkubiert. Das Adsorbermaterial wird dann mit dem 5fachen Spherosilvolumen 0,9 %iger NaCl-Lösung, 3fachen Spherosilvolumen 1M Propionsäure und mit genügend 30 mM NaCl-Lösung gewaschen bis pH 6,5 erreicht ist. Der Adsorber wird in PBS-Puffer pH 7,5 ausreichend äquilibiert.

Durch Chromatographie an Rinderserumalbumin-Biotin- und/oder Streptavidin-Adsorber auf Spherosil-Basis wird inaktiviertes Streptavidin nach Beispiel 3,4 oder 6 von Streptavidin mit verbliebener Restaktivität (Biotinbindung), von verbliebenem freiem Biotin oder von Streptavidin mit kovalentem an der Oberfläche zugänglichem Biotin (im Gegensatz zum in der Bindetasche fixiertem Biotin) gereinigt.

In den Reaktionsansatz wird pro 10 mg Protein 1 ml Streptavidin-Spherosil-Adsorber, äquilibiert in PBS-Puffer, pH 7,5, gegeben und 2 Stunden bei 25°C gerührt.

Die Suspension wird dann in eine Säule überführt und das Säulenmaterial mit PBS-Puffer pH 7,5 gewaschen. Dabei wird am Auslauf der Säule über UV-Monitor bei E₂₈₀ₙₘ der Proteingehalt verfolgt. Es wird bis zur Proteinfreiheit gewaschen. Der proteinhaltige Durchlauf wird in einer Fraktion aufgefangen.

In den proteinhaltigen Durchlauf des Streptavidin-Adsorbers wird dann pro 10 mg Protein 1 ml Rinderserumalbumin-Biotin (RSA-Bi)-Spherosil-Adsorber, äquilibiert in PBS-Puffer, pH 7,5, gegeben und 2 Stunden bei Raumtemperatur gerührt.

Die Suspension wird in eine Säule überführt und mit PBS-Puffer, pH 7,5 gewaschen. Dabei wird am Auslauf der Säule über UV-Monitor bei E₂₈₀ₙₘ der Proteingehalt verfolgt. Der proteinhaltige Durchlauf enthält das Produkt und wird in einer Fraktion aufgefangen. Das Produkt (inaktiviertes (Poly-)Streptavidin oder Thermo-RSA-SA bzw. Derivate und Fragmente) wird auf eine Proteinkonzentration von 20 mg/ml konzentriert und nach Abfüllung lyophilisiert.

### Beispiel 8

### Durchführung eines GAD-Antikörpertests bei sequenzieller Testführung bezüglich biotinyliertem Antigen und Probe

In jede Kavität einer mit ThermoRSA-Streptavidin vorbeschichteten Mikrotiterplatte werden 100 µl biotinylierte Glutamatdecarboxylase (GAD), isoliert aus Schweinegehirn, in einer für die jeweilige Charge optimalen Konzentration (1-3 µg/ml in Inkubationspuffer aus dem Boehringer Mannheim Enzymun-Test® Anti-HIV 1+2) pipettiert und 30 min bei Raumtemperatur inkubiert. Danach wird die Platte dreimal gewaschen mit jeweils 350 µl 50 mmol/L Kaliumphosphat, pH 7,0 mit Zusatz von 0,1 % (w/v) CHAPSO ( 3-[(3-Cholamidopropyl) dimethylammonio]-2-hydroxy-1-propansulfonat). Humanserum wird 1+25 verdünnt in Inkubationspuffer aus dem Enzymun-Test® Anti-HIV 1+2, dem die jeweils angegebene Menge ThermoRSA-Streptavidin (unbehandelt oder inaktiviert) bzw. Streptavidin-Monomer oder Streptavidin-Polymer (unbehandelt oder inaktiviert) zugefügt wurde. Diese so verdünnten Proben werden in einem Volumen von 100 µl/Kavität bei RT mit Schütteln der Mikrotiterplatte 1 Stunde inkubiert. Die Proben werden anschließend abgesaugt und die Platten 3x gewaschen wie oben. Der POD-gekoppelte Nachweisantikörper aus Schaf mit Bindungsspezifität für Human-IgG wird in Kopjugatpuffer aus dem Enzymun-Test® Anti-HIV 1+2 auf eine Konzentration von 75 mU/ml verdünnt und in jeder Kavität 100 µl dieser verdünnten Lösung 1 Stunde bei Raumtemperatur unter Schütteln bei Raumtemperatur inkubiert. Die Flüssigkeit wird abgesaugt und die Platte wieder 3x gewaschen wie oben. Der Farbstoff ABTS® wird zu einer Konzentration von 1 mg/ml in Enzymun-Test® Substratpuffer gelöst und 100 µl/Kavität bei Raumtemperatur ohne Schütteln inkubiert. Nach ca. 30 min wird die Extinktion in einem Mikrotiter-Plattephotometer abgelesen. Meßwellenlänge ist 405 nm und die Referenzwellenlänge 492 nm. Der Leerwert sind zwei unbehandelte Kavitäten, die nur Substrat-/Farbstofflösung enthalten. Der Mittelwert der Extinktion der beiden Leerwert-Kavitäten wird von allen anderen Extinktionen abgezogen.

In den Tabellen 2, 3 und 4 sind jeweils die Mittelwerte von je zwei Kavitäten einer doppelbestimmten Probe in Extinktionen angegeben.

**Tabelle 2**

| **Entstörung durch Zusatz unterschiedlicher Mengen Thermo-RSA-Streptavidin** | | | | | | | | | |
|---|---|---|---|---|---|---|---|---|---|
| **Probe** | **Extinkion ohne Pufferzusatz** | **Signalhöhe in % vom Ausgangssignal bei der jeweiligen Menge Thermo-RSA-SA (% w/v)** | | | | | | | |
| | | 0 | 0,01 | 0,025 | 0,05 | 0,1 | 0,25 | 0,5 | 1 |
| Puffer | 0,016 | * | * | * | * | * | * | * | * |
| Normalserum | 0,182 | 100 | 95 | 68 | 71 | 74 | 70 | 70 | 60 |
| Positivkontrolle (MICA) | 1,906 | 100 | 72 | 74 | 73,5 | 73 | 65 | 62 | 44 |
| Positivserum | 2,065 | 100 | 63 | 61 | 62 | 60 | 50 | 48 | 37 |
| Störserum 1 | 2,325 | 100 | 16 | 14 | 15 | 16 | 15 | 14 | 11 |
| Störserum 2 | 1,243 | 100 | 9 | 8,5 | 8 | 5 | 4 | 4 | 2 |
| Störserum 3 | 0,707 | 100 | 24 | 16 | 13 | 13 | 10 | 10 | 8 |

| | | | | | | | | | |
|---|---|---|---|---|---|---|---|---|---|
| * keine Angabe, da aufgrund des geringen Meßsignales nicht sinnvoll. | | | | | | | | | |
| MICA: monoklonaler Antikörper gegen GAD | | | | | | | | | |

**Tabelle 3**

| **Enstörung durch Zusatz von monomerem oder polymerem Streptavidin** | | | |
|---|---|---|---|
| **Probe** | **ohne Zusatz** | **1% (w/v) SA** | **1% (w/v) PolySA** |
| Puffer | 0,008 | 0,006 | 0,009 |
| Normalserum | 0,106 | 0,144 | 0,108 |
| Störserum | 1,390 | 0,108 | 0,087 |
| Positivkontrolle (MICA) | 0,950 | 1,588 | 1,033 |
| Positivserum | 0,940 | 1,416 | 0,950 |

**Tabelle 4**

| **Entstörung durch Zusatz von Thermo-RSA-Streptavidin oder Thermo-RSA-Streptavidin, das kovalent modifiziert wurde (nach Beispiel 4)** | | | |
|---|---|---|---|
| **Probe** | **ohne Zusatz** | **0,025% (w/v) Thermo-RSA-SA** | **0,025% (w/v) Thermo-RSA-SA(inakt)** |
| Puffer | 0,009 | 0,011 | 0,011 |
| Normalserum | 0,226 | 0,191 | 0,206 |
| Störserum | 1,500 | 0,117 | 0,332 |
| Positivkontrolle (MICA) | 1,745 | 1,528 | 1,579 |
| Positivserum | 1,963 | 1,457 | 0,797 |

### Beispiel 9

### Entstörung eines Anit-HCV-Testes mit Biotin(photoaktiviert)-SA.

### Testprinzip:

2-Schritt-Sandwichassay mit Streptavidinfestphase (Testführung und Reagenzien wie bei Boehringer Mannheim Enzymun-Test® Anti HIV 1+2)
- 1. Schritt:: Biotinylierte Peptide plus Probe
- 2. Schritt:: Reaktion der wandgebundenen Antikörper mit einem Anti-human-IgG-POD Konjugat
- 3. Schritt:: Indikatorreaktion mit ABTS als Substrat

### Puffer:

### a) Inkubationspuffer aus Enzymun-Test® Anti- HIV 1+2

HCV-Peptide aus der Core , NS4 und NS5 Region ± inaktiviertes Streptavidin nach Beispiel 6 und 7 hergestellt

### b) Konjugatpuffer aus Enzymun-Test® Anti- HIV 1+2

### Inkubationszeiten:

- 1. Schritt:: 1 Stunde (Probe + Inkubationspuffer)
- 2. Schritt:: 1 Stunde (+ Konjugatpuffer)
- 3. Schritt:: 1 Stunde (Substratreaktion mit ABTS)

### Proben:

3 negative Serumproben (Referenz 1)
6 falsch positive Anti-HCV-Negativproben
3 positive Anti-HCV-Proben (Referenz 2)

### Volumina:

Probe 20 µl
alle anderen Reagenzien jeweils 500 µl

### Testdurchführung:

am ES 600 bei 25°C nach Testanleitung Enzymun-Test® Anti-HIV 1+2

### Substratmessung:

Messung der Substratlösung bei 422 nm am ES 600 (Boehringer Mannheim GmbH). Die Extinktionen sind in der Tabelle 5 dargestellt.

**Tabelle 5**

| Proben | ohne inakt. SA im Inkubationspuffer | 20 µg/ml inakt. SA im Inkubationspuffer | Signalabnahme nach Zugabe von inakt. SA |
|---|---|---|---|
| Negativserum 1 | 0,036 | 0,027 | * |
| Negativserum 2 | 0,042 | 0,035 | * |
| Negativserum 3 | 0,078 | 0,076 | * |
| HCV-Negativserum 1 | 0,528 | 0,125 | 76% |
| HCV-Negativserum 2 | 0,467 | 0,106 | 77% |
| HCV-Negativserum 3 | 0,979 | 0,161 | 84% |
| HCV-Negativserum 4 | 0,499 | 0,094 | 81% |
| HCV-Negativserum 5 | 2,049 | 0,471 | 77% |
| HCV-Negativserum 6 | 0,427 | 0,065 | 85% |
| HCV Positivserum 1 | 1,747 | 1,645 | 6% |
| HCV Positivserum 2 | 1,161 | 1,076 | 7% |
| HCV Positivserum 3 | 1,104 | 1,026 | 7% |

| | | | |
|---|---|---|---|
| * keine Angabe, da aufgrund des geringen Meßsignales nicht sinnvoll. | | | |

## Patentansprüche

1. Verwendung von Avidin oder Streptavidin oder einem Derivat hiervon als Entstörmittel zur Vermeidung von unspezifischen Wechselwirkungen in Immunoassays oder Nukleinsäureassays.

2. Verwendung von Avidin oder Streptavidin oder einem Derivat hiervon nach Anspruch 1, dadurch gekennzeichnet, daß als Avidin- oder Streptavidinderivat homogen vernetzte Avidin- oder Streptavidinmoleküle eingesetzt werden.

3. Verwendung von Avidin oder Streptavidin oder einem Derivat hiervon nach Anspruch 1, dadurch gekennzeichnet, daß als Avidin oder Streptavidinderivat durch bi- oder polyfunktionelle Verbindungen vernetzte Avidin- oder Streptavidinmoleküle eingesetzt werden.

4. Verwendung von Avidin oder Streptavidin oder einem Derivat hiervon nach Anspruch 1, dadurch gekennzeichnet, daß als Avidin- oder Streptavidinderivat heterogen vernetzte Avidin- oder Streptavidinmoleküle eingesetzt werden.

5. Verwendung von Avidin oder Streptavidin oder einem Derivat hiervon nach Anspruch 4, dadurch gekennzeichnet, daß mit Proteinen vernetzte Avidin- oder Streptavidinmoleküle eingesetzt werden.

6. Verwendung von Avidin oder Streptavidin oder einem Derivat hiervon nach Anspruch 5, dadurch gekennzeichnet, daß mit polymerisierten Proteinen vernetzte Avidin- oder Streptavidinmoleküle eingesetzt werden.

7. Verwendung von Avidin oder Streptavidin oder einem Derivat hiervon nach Anspruch 5 oder 6, dadurch gekennzeichnet, daß als Avidin- oder Streptavidinmoleküle homogen vernetzte Avidin- oder Streptavidinmoleküle eingesetzt werden.

8. Verwendung von Avidin oder Streptavidin oder einem Derivat hiervon nach Anspruch 1, dadurch gekennzeichnet, daß als Avidin oder Streptavidinderivat Fragmente, die in der Lage sind, die Entstörwirkung des gesamten Moleküls beizubehalten, oder ein Gemisch aus diesen Fragmenten aus Avidin oder Streptavidin eingesetzt werden.

9. Verwendung von Avidin oder Streptavidin oder einem Derivat hiervon nach Anspruch 1 bis 8 dadurch gekennzeichnet, daß als Avidin- oder Streptavidinderivat inaktiviertes Avidin oder Streptavidin eingesetzt wird.

10. Verwendung von Avidin oder Streptavidin oder einem Derivat hiervon nach Anspruch 9, dadurch gekennzeichnet, daß die Inaktivierung von Avidin oder Streptavidin durch Absättigung mit Biotin oder einem Biotinderivat erfolgte.

11. Verwendung von Avidin oder Streptavidin oder einem Derivat hiervon nach Anspruch 9, dadurch gekennzeichnet, daß die Inaktivierung durch kovalente Modifizierung des aktiven Zentrums von Avidin oder Streptavidin erfolgte.

12. Verwendung von Avidin oder Streptavidin oder einem Derivat hiervon nach Anspruch 11, dadurch gekennzeichnet, daß zur kovalenten Modifizierung eine Derivatisierung mindestens einer Aminosäure des aktiven Zentrums oder eine kovalente Kopplung von Biotin ans aktive Zentrum durchgeführt wird.

13. Verwendung von Avidin oder Streptavidin oder einem Derivat hiervon nach Anspruch 12, dadurch gekennzeichnet, daß die kovalente Kopplung von Biotin ans aktive Zentrum durch photoaktivierbares Biotin, beispielsweise Biotin-DADOO-AB, erfolgt.

14. Verwendung von Avidin oder Streptavidin oder einem Derivat hiervon nach Anspruch 9, dadurch gekennzeichnet, daß die Desaktivierung des aktiven Zentrums durch gentechnologische Methoden, wie Substitution, Deletion oder Insertion einzelner oder mehrerer Aminosäurereste erfolgt.

15. Verwendung von Avidin oder Streptavidin oder einem Derivat hiervon nach einem der Ansprüche 9 - 14, dadurch gekennzeichnet, daß das inaktivierte Avidin oder Streptavidin zusätzlich über festphasengebundenes Biotin und/oder Avidin oder Streptavidin gereinigt wurde.

16. Verwendung eines Entstörmittels nach einem der Ansprüche 1 bis 15 in einem Immunoassay oder einem Nukleinsäureassay, bei dem als eine Bindungskomponente Avidin oder Streptavidin eingesetzt wird.

17. Verfahren zur Bestimmung eines Analyten in einer Probe umfassend die Schritte
(1) Kontaktieren der Probe mit
(a) einem Entstörmittel nach einem der Ansprüche 1 bis 15
(b) einem oder mehreren spezifischen Bindepartnern des Analyten und
(2) Messung des gebildeten Komplexes aus Analyt und spezifischem Bindepartner als ein Maß für die Anwesenheit des Analyten.

18. Verfahren zur Bestimmung eines Analyten in einer Probe nach Anspruch 17, dadurch gekennzeichnet, daß mindestens eine Bindekomponente des Verfahrens über Avidin/Biotin oder Streptavidin/Biotin gekoppelt wird und die Kopplung dieser Bindekomponente vor der Zugabe des Entstörmittels erfolgt.

19. Verfahren zur Bestimmung eines Analyten in einer Probe umfassend die Schritte
(1) Kontaktieren der Probe mit
(a) einem Entstörmittel nach einem der Ansprüche 1 - 15
(b) einem oder mehreren spezifischen Bindepartnern des Analyten und
(2) Messung des gebildeten Komplexes aus Analyt und spezifischem Bindepartner als ein Maß für die Anwesenheit des Analyten, wobei das Kontaktieren der Probe mit (a) und (b) gleichzeitig erfolgt.

20. Testkombination für ein Verfahren zur Bestimmung eines Analyten in einer Probe enthaltend
(1) ein Avidin- oder Streptavidinderivat, das nicht in den Komplex aus Analyt und Analytbindungspartner eingebaut wird nach einem der Ansprüche 1 bis 15 und
(2) mindestens einen spezifischen Bindepartner des Analyten.

21. Testkombination nach Anspruch 20 enthaltend zusätzlich alle weiteren für das Verfahren zur Bestimmung notwendigen Reagenzien.

22. Verfahren zur Herstellung eines Entstörmittels nach einem der Ansprüche 9 bis 15, dadurch gekennzeichnet, daß Avidin oder Streptavidin modifiziert und gegebenenfalls über festphasengebundenes Biotin und/oder Avidin- oder Streptavidin gereinigt wird.

23. Inaktiviertes Avidin oder Streptavidin, erhältlich durch Absättigung der aktiven Zentren durch Biotin oder einem Biotinderivat oder kovalente Modifizierung des aktiven Zentrums und Aufreinigung über festphasengebundenes Biotin und/oder Avidin- oder Streptavidin.

24. Inaktiviertes Avidin oder Streptavidin, bei dem Biotin an das aktive Zentrum und zusätzlich über eine kovalente Bindung außerhalb des aktiven Zentrums gebunden ist, erhältlich durch Absättigung der aktiven Zentren von Avidin oder Streptavidin mit einem photoaktivierbaren Biotinderivat und anschließender kovalenter Kopplung des Biotinderivats durch Initiieren der Photoreaktion.

25. Inaktiviertes Avidin oder Streptavidin nach Anspruch 24, dadurch gekennzeichnet, daß als Biotinderivat Biotin-DADOO-AB verwendet wird.

## Claims

1. Use of avidin or streptavidin or a derivative thereof as an interference-eliminating agent to avoid unspecific interactions in immunoassays or nucleic acid assays.

2. Use of avidin or streptavidin or a derivative thereof as claimed in claim 1, wherein homogeneously cross-linked avidin or streptavidin molecules are used as the avidin or streptavidin derivative.

3. Use of avidin or streptavidin or a derivative thereof as claimed in claim 1, wherein avidin or streptavidin molecules cross-linked by bifunctional or polyfunctional compounds are used as the avidin or streptavidin derivative.

4. Use of avidin or streptavidin or a derivative thereof as claimed in claim 1, wherein heterogeneously cross-linked avidin or streptavidin molecules are used as the avidin or streptavidin derivative.

5. Use of avidin or streptavidin or a derivative thereof as claimed in claim 4, wherein avidin or streptavidin molecules cross-linked with proteins are used.

6. Use of avidin or streptavidin or a derivative thereof as claimed in claim 5, wherein avidin or streptavidin molecules cross-linked with polymerized proteins are used.

7. Use of avidin or streptavidin or a derivative thereof as claimed in claim 5 or 6, wherein homogeneously cross-linked avidin or streptavidin molecules are used as the avidin or streptavidin molecules.

8. Use of avidin or streptavidin or a derivative thereof as claimed in claim 1, wherein fragments that are able to retain the interference-eliminating effect of the whole molecule or a mixture of these fragments of avidin or streptavidin are used as the avidin or streptavidin derivative.

9. Use of avidin or streptavidin or a derivative thereof as claimed in claims 1 to 8, wherein inactivated avidin or streptavidin is used as the avidin or streptavidin derivative.

10. Use of avidin or streptavidin or a derivative thereof as claimed in claim 9, wherein avidin or streptavidin is inactivated by saturation with biotin or a biotin derivative.

11. Use of avidin or streptavidin or a derivative thereof as claimed in claim 9, wherein the inactivation is achieved by covalently modifying the active centre of avidin or streptavidin.

12. Use of avidin or streptavidin or a derivative thereof as claimed in claim 11, wherein for the covalent modification at least one amino acid of the active centre is derivatized or biotin is covalently coupled to the active centre.

13. Use of avidin or streptavidin or a derivative thereof as claimed in claim 12, wherein biotin is covalently coupled to the active centre by photo-activatable biotin, for example biotin-DADOO-AB.

14. Use of avidin or streptavidin or a derivative thereof as claimed in claim 9, wherein the active centre is deactivated by genetic engineering methods such as substitution, deletion or insertion of individual or several amino acid residues.

15. Use of avidin or streptavidin or a derivative thereof as claimed in one of the claims 9 - 14, wherein the inactivated avidin or streptavidin has been additionally purified over solid phase-bound biotin and/or avidin or streptavidin.

16. Use of an interference-eliminating agent as claimed in one of the claims 1 to 15 in an immunoassay or a nucleic acid assay in which avidin or streptavidin is used as a binding component.

17. Method for the determination of an analyte in a sample comprising the steps
(1) contacting the sample with
(a) an interference-eliminating agent as claimed in one of the claims 1 to 15
(b) one or several specific binding partners of the analyte and
(2) measuring the complex formed from the analyte and specific binding partner as a measure for the presence of the analyte

18. Method for the determination of an analyte in a sample as claimed in claim 17, wherein at least one binding component of the method is coupled via avidin/biotin or streptavidin/biotin and the coupling of this binding component is carried out before addition of the interference-eliminating agent.

19. Method for the determination of an analyte in a sample comprising the steps
(1) contacting the sample with
(a) an interference-eliminating agent as claimed in one of the claims 1 - 15
(b) one or several specific binding partners of the analyte and
(2) measuring the complex formed from analyte and specific binding partner as a measure for the presence of the analyte in which the sample is simultaneously contacted with (a) and (b).

20. Test combination for a method for determining an analyte in a sample containing
(1) an avidin or streptavidin derivative as claimed in one of the claims 1 to 15 which is not incorporated into the complex formed from analyte and analyte binding partner and
(2) at least one specific binding partner of the analyte.

21. Test combination as claimed in claim 20 additionally containing all further reagents required for the determination method.

22. Process for the production of an interference-eliminating agent as claimed in one of the claims 9 to 15, wherein avidin or streptavidin is modified and optionally purified over solid phase-bound biotin and/or avidin or streptavidin.

23. Inactivated avidin or streptavidin obtainable by saturating the active centres with biotin or a biotin derivative or by covalent modification of the active centre and purification over solid phase bound biotin and/or avidin or streptavidin.

24. Inactivated avidin or streptavidin in which biotin is bound to the active centre and is additionally bound outside the active centre via a covalent bond obtainable by saturating the active centres of avidin or streptavidin with a photoactivatable biotin derivative and subsequently covalently coupling the biotin derivative by initiating the photoreaction.

25. Inactivated avidin or streptavidin as claimed in claim 24, wherein biotin-DADOO-AB is used as the biotin derivative.

## Revendications

1. Utilisation d'avidine ou de streptavidine ou d'un dérivé de celles-ci à titre d'agent antiparasite pour éviter des interactions non-spécifiques dans les dosages immunologiques ou dans les dosages d'acides nucléiques.

2. Utilisation d'avidine ou de streptavidine ou d'un dérivé de celles-ci selon la revendication 1, caractérisée en ce qu'on utilise à titre de dérivé d'avidine ou de streptavidine des molécules d'avidine ou de streptavidine réticulées de manière homogène.

3. Utilisation d'avidine ou de streptavidine ou d'un dérivé de celles-ci selon la revendication 1, caractérisée en ce qu'on utilise à titre de dérivé d'avidine ou de streptavidine des molécules d'avidine ou de streptavidine réticulées par des composés bi- ou polyfonctionnels.

4. Utilisation d'avidine ou de streptavidine ou d'un dérivé de celles-ci selon la revendication 1, caractérisée en ce qu'on utilise à titre de dérivé d'avidine ou de streptavidine des molécules d'avidine ou de streptavidine réticulées de manière hétérogène.

5. Utilisation d'avidine ou de streptavidine ou d'un dérivé de celles-ci selon la revendication 4, caractérisée en ce qu'on utilise des molécules d'avidine ou de streptavidine réticulées avec des protéines.

6. Utilisation d'avidine ou de streptavidine ou d'un dérivé de celles-ci selon la revendication 5, caractérisée en ce qu'on utilise des molécules d'avidine ou de streptavidine réticulées avec des protéines polymérisées.

7. Utilisation d'avidine ou de streptavidine ou d'un dérivé de celles-ci selon la revendication 5 ou 6, caractérisée en ce qu'on utilise à titre de dérivé d'avidine ou de streptavidine des molécules d'avidine ou de streptavidine réticulées de manière homogène.

8. Utilisation d'avidine ou de streptavidine ou d'un dérivé de celles-ci selon la revendication 1, caractérisée en ce qu'on utilise à titre de dérivé d'avidine ou de streptavidine des fragments qui sont capables de conserver l'action antiparasite de la molécule entière, ou un mélange de ces fragments d'avidine ou de streptavidine.

9. Utilisation d'avidine ou de streptavidine ou d'un dérivé de celles-ci selon la revendication 1 à 8, caractérisée en ce qu'on utilise à titre de dérivé d'avidine ou de streptavidine de l'avidine ou de la streptavidine inactivée.

10. Utilisation d'avidine ou de streptavidine ou d'un dérivé de celles-ci selon la revendication 9, caractérisée en ce que l'inactivation de l'avidine ou de la streptavidine a été effectuée par saturation par la biotine ou par un dérivé de biotine.

11. Utilisation d'avidine ou de streptavidine ou d'un dérivé de celles-ci selon la revendication 9, caractérisée en ce que l'inactivation a été effectuée par la modification covalente du centre actif de l'avidine ou de la streptavidine.

12. Utilisation d'avidine ou de streptavidine ou d'un dérivé de celles-ci selon la revendication 11, caractérisée en ce qu'on effectue, en vue de la modification covalente, une dérivatisation d'au moins un acide aminé du centre actif ou un couplage covalent de la biotine au centre actif.

13. Utilisation d'avidine ou de streptavidine ou d'un dérivé de celles-ci selon la revendication 12, caractérisée en ce que le couplage covalent de la biotine au centre actif est effectué par de la biotine photoactivable, par exemple la biotine-DADOO-AB.

14. Utilisation d'avidine ou de streptavidine ou d'un dérivé de celles-ci selon la revendication 9, caractérisée en ce que l'inactivation du centre actif a été effectuée par des méthodes de génie génétique, telles que par substitution, par délétion ou par insertion d'un ou de plusieurs résidus d'acide aminé.

15. Utilisation d'avidine ou de streptavidine ou d'un dérivé de celles-ci selon l'une des revendications 9 à 14, caractérisée en ce que l'avidine ou la streptavidine inactivée a été purifiée davantage par le passage sur de la biotine et/ou de l'avidine ou de la streptavidine liée à une phase fixe.

16. Utilisation d'un agent antiparasite selon l'une des revendications 1 à 15 dans un dosage immunologique ou dans un dosage d'acides nucléiques, dans lequel on utilise à titre de composant de liaison de l'avidine ou de la streptavidine.

17. Procédé pour la détermination d'un analyte dans un échantillon comprenant les étapes de
(1) mise en contact de l'échantillon avec
(a) un agent antiparasite selon l'une des revendications 1 à 15
(b) un ou plusieurs partenaires de liaison spécifiques de l'analyte et
(2) la mesure du complexe formé à partir de l'analyte et du partenaire de liaison spécifique à titre de mesure de la présence de l'analyte.

18. Procédé pour la détermination d'un analyte dans un échantillon selon la revendication 17, caractérisé en ce qu'au moins un composant de liaison du procédé est couplé par l'avidine/biotine ou la streptavidine/biotine et en ce que le couplage de ce composant de liaison est effectué avant l'addition de l'agent antiparasite.

19. Procédé pour la détermination d'un analyte dans un échantillon comprenant les étapes de
(1) mise en contact de l'échantillon avec
(a) un agent antiparasite selon l'une des revendications 1 à 15
(b) un ou plusieurs partenaires de liaison spécifiques de l'analyte et
(2) la mesure du complexe formé à partir de l'analyte et du partenaire de liaison spécifique à titre de mesure de la présence de l'analyte, la mise en contact de l'échantillon avec (a) et avec (b) étant réalisée simultanément.

20. Combinaison de test pour un procédé de détermination d'un analyte dans un échantillon, contenant
(1) un dérivé d'avidine ou de streptavidine qui n'est pas intégré dans le complexe constitué de l'analyte et du partenaire de liaison de l'analyte selon l'une des revendications 1 à 15, et
(2) au moins un partenaire de liaison spécifique de l'analyte.

21. Combinaison de test selon la revendication 20, contenant en outre tous les autres réactifs nécessaires au procédé de détermination.

22. Procédé de préparation d'un agent antiparasite selon l'une des revendications 9 à 15, caractérisé en ce que l'avidine ou la streptavidine est modifiée et éventuellement purifiée par le passage sur de la biotine et/ou de l'avidine ou de la streptavidine liée à une phase fixe.

23. Avidine ou streptavidine inactivée, obtenue par saturation des centres actifs par la biotine ou un dérivé de biotine ou par la modification covalente du centre actif et la purification sur de la biotine et/ou de l'avidine ou de la streptavidine liée à une phase fixe.

24. Avidine ou streptavidine inactivée, dans laquelle de la biotine est liée au centre actif et qui est en outre liée par une liaison covalente à l'extérieur du centre actif, obtenue par saturation des centres actifs de l'avidine ou de la streptavidine avec un dérivé de biotine photoactivable et par le couplage covalent subséquent du dérivé de biotine par l'initiation de la photoréaction.

25. Avidine ou streptavidine inactivée selon la revendication 24, caractérisée en ce qu'on utilise à titre de dérivé de biotine la biotine-DADOO-AB.
